(19) **Europäisches Patentamt**
**European Patent Office**
**Office européen des brevets**

(11) **EP 3 190 986 B1**

(12) **EUROPEAN PATENT SPECIFICATION**

(45) Date of publication and mention
of the grant of the patent:
**22.05.2019 Bulletin 2019/21**

(21) Application number: **15778722.7**

(22) Date of filing: **03.09.2015**

(51) Int Cl.:
*A61B 17/12* *(2006.01)*    *A61C 13/15* *(2006.01)*

(86) International application number:
**PCT/IB2015/056718**

(87) International publication number:
**WO 2016/038515 (17.03.2016 Gazette 2016/11)**

(54) **DEVICE FOR PHOTOACTIVATION AND REACTION MONITORING**

VORRICHTUNG ZUR PHOTOAKTIVIERUNG UND REAKTIONSBEOBACHTUNG

DISPOSITIF DE PHOTO-ACTIVATION ET SURVEILLANCE DE RÉACTIONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.09.2014 US 201462047095 P**

(43) Date of publication of application:
**19.07.2017 Bulletin 2017/29**

(73) Proprietor: **Ecole Polytechnique Fédérale de
Lausanne (EPFL)
1015 Lausanne (CH)**

(72) Inventors:
• **SCHMOCKER, Andreas
1007 Lausanne (CH)**
• **MOSER, Christophe
1007 Lausanne (CH)**

• **PIOLETTI, Dominique
1164 Buchillon (CH)**
• **BOURBAN, Pierre-Etienne
1260 Nyon (CH)**

(74) Representative: **Grosfillier, Philippe
ANDRE ROLAND S.A.
P.O. Box 5107
1002 Lausanne (CH)**

(56) References cited:
**EP-A1- 1 236 444     EP-A1- 1 835 278
EP-A1- 1 923 018     EP-A1- 2 529 690
EP-A2- 1 970 696     US-A1- 2006 240 376
US-A1- 2007 259 309  US-A1- 2008 305 459
US-A1- 2012 077 895**

## Description

FIELD OF THE INVENTION

The present invention generally relates to photopolymerization devices and more particularly to devices that use light to harden, activate, control or change the chemical and physical state of a photo-chemically active or responsive material.

BACKGROUND

Medical implants are used to replace impaired parts of the human or animal body. Usually a preformed material is placed inside the body and mechanically fixed to bone or soft tissue. In most cases such interventions are highly invasive. One option to reduce this invasiveness is to inject the entire implant (or a part of it) in liquid form and then harden the material *in situ* by a chemical reaction. However, these chemical reactions are usually hard to control, often impossible to stop, heat up surrounding tissue or require toxic additives. To sidestep such issues photo-active materials such as photopolymers are an attractive solution. Once injected they are illuminated with light in the visible or ultraviolet spectrum. The absorbed photons change the energy levels of electrons which then trigger the chemical reaction by creating free radicals, cations or anions. At the same time a fraction of the electrons relaxes back to the ground state, hence creating fluorescence which spectrum and intensity indicates the state of the chemical reaction.

However, to place such materials in a minimally invasive way it is necessary to access the body part with an injective device, a light source and a monitoring device. The current injection devices cannot be integrated into an illumination and monitoring system or vice versa. All-in-one integrated and ergonomic systems with a small diameter (e.g less than one 1mm) are required. Moreover, methods have to be developed to ensure maximal adherence of implanted polymer to native tissue. Document US2006/240376 discloses a system according to the preamble of claim 1.

SUMMARY OF THE INVENTION

The present invention is disclosed in appended claim 1. The present invention relates to a device structure and physical apparatus to photopolymerize or activate a photosensitive material and to monitor or control this process, using actinic light and a coupled analysis system which analyzes light coming from the photosensitive material to determine the degree of photopolymerization or activation of the photosensitive material during the photoactivation process.

In one aspect, the invention provides for an optical device, wherein said device is designed to interact with a photosensitive and/or a photocurable material, and wherein said device comprises a light source that emits actinic light, a tubular applicator having a proximal end and a distal end and an elongated shaft therebetween and containing at least one light-transmitting element adapted to bidirectionally transmit light between said proximal end and said distal end, wherein said proximal end of the applicator is operably connected to the light source; and wherein the distal end of the applicator is arranged to emit actinic light originated from the light source to the photocurable material and to capture light reflected or emitted by the photocurable material; and a light-guiding element which directs light travelling from the distal end of the applicator through the at least one light-transmitting element towards an optical detector, said optical detector being capable of detecting the light reflected or emitted by the photocurable material.

The apparatus according to the present disclosure comprises an optical system having one or several light sources, light-transmitting elements such as optical wave guides, light-guiding elements such as mirrors and/or beam combiners, free space and/or other optical subsystems to guide the light to the material to be illuminated. This optical system does not only guide the light to the material, but also collects the reflected, emitted or backscattered light from the material for monitoring purposes. Thus, the optical system also includes a subsystem to guide and finally evaluate such reflected/backscattered light. It consists of a dichroic beam-splitter and/or filter to separate the illumination and reflected/backscattered light, especially if the reflected/backscattered light is collected at the same position as the material is illuminated. Yet, it can also be completely separate e.g. consisting of a separate waveguide to collect the reflected and backscattered light.

In a preferred aspect, the light-transmitting elements of the illumination and monitoring device consist of optical fibers.

In one aspect, the light-guiding element includes beam splitter, band-pass filter and Bragg grating.

In one aspect, the light source emits light within a wavelength range of 200 - 3000 nanometers, preferably in a range of 200 - 700 nanometers, more preferably in a range of 315 - 700 nanometers.

The optical system also contains an analysis subsystem that may contain for instance a spectrometer to quantify fluorescence or Raman scattering or other types of back-reflected or back-scattered or emitted light, indicative of the state of the chemical or photochemical reaction.

The light applied to and collected from the photosensitive material travels through an applicator having an elongated, tubular structure which allows photoactivation of materials on surfaces, in cavities, hollow recipients, tissues and within living organisms. In certain embodiments, the applicator is a cannula or a catheter containing optical fibers, connected to a light source and a spectrometer. Because the device is capable of characterizing the light-induced transformation of the photosensitive material in real time, optimal exposure of the photosensitive material to actinic light can be achieved, for example to obtain an optimal degree of polymerization and thus the best possible physical properties of a photopolymerized material for a specific purpose.

Another aspect of the invention lies in the combination of the illuminating and monitoring system with an injection system, allowing deposition of photosensitive material, illumination and monitoring through one single applicator. In addition to one or more light transmitting elements, such an applicator contains at least one channel through which a fluid photocurable or otherwise photosensitive material can be injected or deposited at a target site such as a cavity or a living organism's tissue. Therefore, in a further aspect, the invention provides for a system comprising an optical device as disclosed above, wherein the applicator is a tubular element having a wall and a lumen, and comprising at least one light-transmitting element placed within the lumen of the tubular element, and at least one interspace between said light-transmitting element and the internal side of the wall of said tubular element allowing the delivery of a photocurable fluid material through the distal end of the applicator into or onto a cavity or a tissue of a living host. In this context, the interspace can comprise a further injection device or at least a portion thereof; in an alternative or additional embodiment, the interspace coaxially surrounds the light-transmitting element, thus rendering the applicator itself a portion of the injection device. In an alternative or additional embodiment, the light-transmitting element and/or the injection device is incorporated within the applicator's wall. As will be evident to a person skilled in the relevant art, an injection device can comprise any kind of suitable pressure sources in order to apply a positive pressure on the liquid to be injected.

In the present application, the device of the invention further comprises a subsystem to introduce one or more fluids to the interspace between the light-transmitting element and the wall of the tubular element at or close to the applicator's proximal end, said fluids once mixed constituting a photocurable fluid destined to be applied into or onto a cavity or a tissue of a living host. In addition, the photocurable material can be put and possibly held under pressure in order to increase adherence to the surrounding tissue or cavity wall.

In a preferred aspect, the applicator is a needle, a cannula, a catheter or an endoscopic arm.

In a preferred aspect, the photocurable material is a material that, once photocured, transforms from a fluid pre-polymeric condition to a polymeric, non-fluent condition.

In one aspect the intensity and illumination time of the light is adapted to affect (e.g. photocure) only injected material at a certain distance of the distal end. Thus, creating a controlled illuminated volume where injected material which is situated outside of this volume is not affected and can be, for instance, leave the body through the cardiovascular system. In addition such a volume can be further controlled during a surgery, by injecting and illuminating material in several steps and/or moving the optical light guide. Moreover, it can be combined with or consist of other elements limiting the light propagation and thus further controlling the volume activated by light. Such an element might be an additionally added element (such as a balloon) or an element which is part of the host (e.g. tissue wall).

It is among the general objects of the invention to also provide for techniques to effectively and efficiently applying a fluent polymerizable material to a target site, including living hosts' tissues, and for effecting polymerization of the fluent light-sensitive material in situ in an optimal way to obtain a desired degree of polymerization conferring to the applied material the best possible physical and/or chemical properties. To achieve such an optimal photopolymerization, the photopolymerization process is monitored and, for example, application of actinic light is stopped when the light emitted or reflected by the photopolymerized material indicates that the desired degree of polymerization has been reached.

In addition, the light reflected by the photopolymerized material can not only indicate the material properties directly at the tip, but also at a certain depth within the material. For instance it can distinguish between whether the tip is surrounded by injected, photo-active material, by blood or by tissue. Or, if it is directly surrounded by injected material, but behind the material is tissue, the signal will vary depending on the distance the light travels through the injected material. Base on the signal the distance between probe and tissue or the thickness of the applied, injected material can be estimated.

In a further aspect, the present disclosure relates to a method of applying, photocuring and monitoring a material into or onto a tissue or cavity, the method comprises applying from the applicator of the previously described system an initially entirely fluent, pre-polymeric photocurable material to the tissue or cavity, applying actinic light through at least one light-transmitting element to the photocurable material for a period of time sufficient to convert the entirely fluent, pre-polymeric photocurable material to a polymeric, non-fluent material, the polymeric, non-fluent material being in an amount effective to cover at least a portion of the target tissue and monitoring the curing process, wherein the initially entirely fluent, pre-polymeric photocurable material is applied into or onto the tissue or cavity through release from the distal end of said applicator, and wherein the at least one light-transmitting element further capture light reflected or emitted by the applied photocurable material and deliver said reflected or emitted light to a light-guiding element which directs light travelling from the distal end towards an optical detector, said optical detector being capable of detecting the light reflected or emitted by the photocurable material, and wherein monitoring the curing process involves analyzing a change in the properties of the light reflected or emitted by the photocurable material and detected by the optical detector, said change being a direct indication of the photocuring process itself.

When applied on living organisms such as animals, including human beings, a particular aspect of the invention relies in a method of replacing, healing or otherwise treating a damaged or altered organ or tissue in a living host by precisely injecting a photosensitive material, preferably in a minimally invasive way, to a target body site through the above-described method.

In one aspect, therefore, the tissue or cavity is a body tissue or body cavity. In a preferred aspect, the body tissue or body cavity is from an animal, including human beings. In a particular aspect, the method further comprises the step of introducing the applicator inside the animal body through surgical means or through an orifice.

In one aspect, the light delivery system is also used for imaging of the tissue.

In one aspect, the photocurable material is an implant, filler, tissue replacement, gel or scaffold applied to a living host. In a preferred aspect, the photocurable material is a biomaterial such as photo-responsive hydrogels (containing e.g. Polyethylen Glycol, Hyaluronans, methacrylates and the like), composite hydrogel (including e.g. cellulose fiber), gelatin-agar system, gel based on amino acids sequences derived from proteins, collagen, silk fibers, polyurethane, cellulose, poly vinyl alcohol or other poly- or copolymers, or curable or crosslinkable material.

In one aspect, photosensitizers sensible in the visible wavelength such as Riboflavin, Rose Bengal or Camphorquinone are used to induce the photochemical reaction. In another aspect photosensitizers in the ultraviolet range such as Irgacure 819 or Irgacure 2959 are used.

In one aspect a contrast agent such as Iodine based agents or other agents used in clinics for fluoroscopy, CT-scans or X-ray imaging is mixed to the injected material which allows to image the injected volume from outside of the animal or human body. Thus, the exact position of the material can be identified. Furthermore, leaking material can be traced or holes in tissue or bone can be closed (closed meaning that there is no leakage) by illuminating the material at the position where it leaks.

In at least one embodiment, the device of the invention are used for treatment or prevention of a pathological condition or for cosmetic procedures.

In at least one embodiment, the device of the invention are used to replace completely or partly an organ such as part of the intervertebral disc.

In at least one embodiment, the device of the invention are used to replace, heal or strengthen cartilage tissues such as the articular cartilage of any joints or non-hyaline cartilage.

In at least one embodiment, the device of the invention are used in dental applications such as for instance the injection and hardening of dental cement or hydrogels/composite hydrogels in a minimally invasive way.

In at least one embodiment, the device of the invention are used for injection and photopolymerization of materials to treat aneurysms.

In at least one embodiment, the device of the invention are used for cosmetic and esthetic surgery procedures. This could be augmentation mammoplasty or a treatment of glabellar lines by an injection similar to a treatment with Botulinum toxin A or hydrogel.

In at least one aspect to the disclosure relates to inject, fix or otherwise position a photosensitive material comprising a drug or a pro-drug into or onto a cavity or a tissue in a controlled manner through the device of the invention. This could be for instance surgical methods to treat e.g. cancer where a material containing a (pro-)drug is placed close or into the cancerous tissue. The illumination with actinic light provided by the device is used to fix the material at a given location. In another embodiment the light photoactivates the drug as for example in phototherapy.

BRIEF DESCRIPTION OF THE FIGURES

     Fig. 1 is a conceptual overview of the device and its subsystems.
     Fig. 2 is a conceptual view of the optical subsystems for illumination and monitoring.
     Fig. 3 illustrates the phenomena underlying the online data analysis, including change in spectra due to photopolymerization and comparison to photorheology data.
     Fig. 4 depicts the combination of injection system and optical light guide in a preferred embodiment allowing for the mixing of two fluid materials and the flow of these materials along an optical fiber. Solid arrows indicate flow of liquid material; dashed arrows indicate light propagation.
     Fig. 5 illustrates the insertion of the tip of the applicator into a cavity or tissue. Fine solid arrows indicate material flow; dashed arrows indicate light propagation, absorption and scattering.
     Fig. 6 illustrates the flow locking system of some embodiments.
     Fig. 7 Illustrates how the injection and photopolymerization method may increase adherence of a photopolymerizable material to a cavity.
     Fig. 8 Illustrates how a cavity is created artificially.

DETAILED DESCRIPTION OF THE INVENTION

The present disclosure may be more readily understood by reference to the following detailed description presented in connection with the accompanying drawing figures, which form a part of this disclosure. It is to be understood that this disclosure is not limited to the specific conditions or parameters described and/or shown herein, and that the terminology used herein is for the purpose of describing particular embodiments by way of example only and is not intended to be limiting of the claimed disclosure.

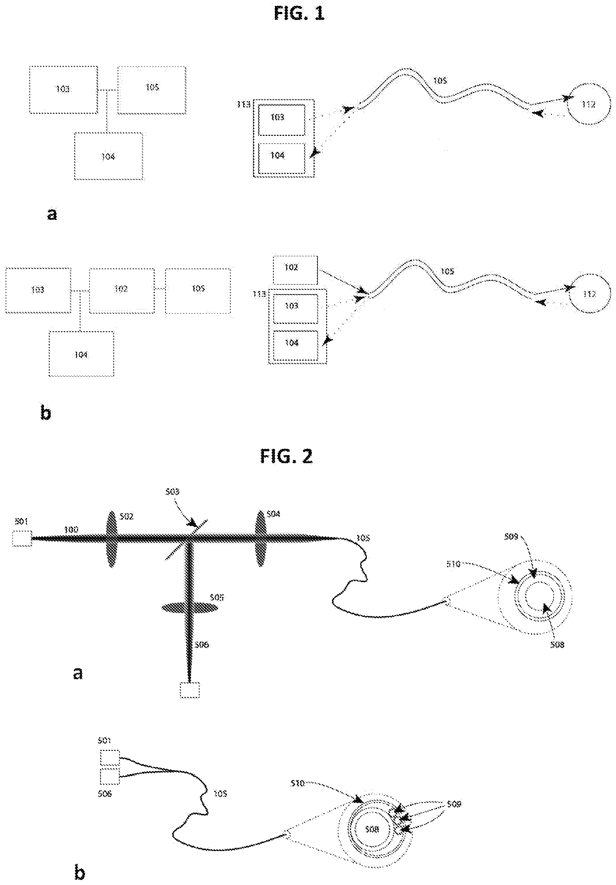

FIG. 1 is an illustration of two possible embodiments of a device according to the present disclosure. Fig. 1a illustrates a schematic overview of a device comprising at least two functionalities: illumination of a material deposited in a cavity or body tissue 112, and analysis of light that is reflected by or emitted by the illuminated material. To achieve this, the device comprises 3 functional units: an illumination system 103 capable of emitting actinic light, an optical detection and data analysis system 104 suited to analyze light coming from the illuminated material, and a system 105, termed applicator, that allows the targeted application and collection of light to or from a material deposited in a cavity or body tissue. The device consists of at least 2 physical subsystems including a casing 113 comprising a source of actinic light 103 and an optical detection and data analysis system 104; and an applicator 105 that combines the possibility to deliver actinic light to a cavity or body tissue 112 in order to activate or photocure a suitable applied photosensitive or photocurable material and to collect light that is reflected, backscattered or emitted by the material illuminated with actinic light. The light coming from the illuminated material is guided to the optical detection and data analysis system 104 allowing real time monitoring of the photoactivation or photocuring process. The terms "photoactivation" refers to a chemical change a photosensitive material undergoes when illuminated with actinic light, said chemical change providing said material with one or more new functionalities. The terms "photocuring", "curing", "photopolymerization", and the like are herein used interchangeably and refer to the toughening or hardening of a material by crosslinking of polymer chains or to the formation of a new polymer-based material by linking several monomers. Such photocurable materials are polymerized upon exposure to electromagnetic radiation, i.e., light. The light may be microwave, infrared, visible, or ultraviolet light, most typically visible or near ultraviolet. "Actinic light" refers to the kind of light to which a particular photosensitive material is sensitive; in other words, actinic light has the capacity to activate, polymerize or somehow alter the properties of a particular photosensitive material. The term "reflection" or "backscattering" includes specular and diffuse reflection including backscattering. The term "reflected" or "backscattered" refers to radiation reflected by specular or diffuse reflection including backscattering. Specular meaning that the reflected light can have a different wavelength than the illumination light such as for instance for Raman scattering or the emission of fluorescence or phosphorescence.

The applicator 105 has an elongated shape with a proximal end and a distal end. In the frame of the present disclosure, the word "applicator" refers to any tool or device used to apply an actinic light directly into or onto an area of interest. In at least some aspects, the applicator is a tubular element comprising at least one light-transmitting element such as for instance optical fibers. In one embodiment, the applicator consists of one or more light-transmitting elements which are designed to supply actinic light to the distal end of the applicator and to transmit returning light. In an alternative embodiment of the invention, the applicator has a body, connecting the proximal and distal ends, defining a lumen which contains the light-transmitting elements. The term "distal" refers to a direction toward the end of the device near where the light interacts with the photosensitive material; the term "proximal" refers to the opposite direction, that is, toward the optical detection and data analysis system. The body typically has a length between 0.5 and 500 cm. The body typically has a wall, which is usually made of a biocompatible and resilient metal. The wall is typically constructed from nitinol or stainless steel. In some embodiments, the applicator wall may be a commercially available syringe needle, catheter or a cannula. In yet another embodiment, the applicator may be an endoscope. In some embodiments, the distal end of the applicator may contain a needle. The light-transmitting elements contained in the applicator according to this embodiment of the invention may fill the entire lumen of the body of the applicator, or they may be bound to the wall with adhesive or fasteners or may be touching the wall or may be displaced axially from the wall with spacers, typically made from a resilient polymer. In some particular embodiments, the body of the applicator consists of a catheter or any suitable tubular element having a shape adaptable to the area within which it is inserted, such as for instance a blood vessel. An applicator according to this aspect of the invention may be of various size and shape, typically has a tubular shape, and is constructed of a soft, flexible, biocompatible material.

The system for illumination 103 of the subsystem 113 may comprise any known light sources capable of producing light with the desired temporal and frequency characteristics. System for illumination 103 may be, for example, solid-state lasers, gas lasers, dye lasers, or semiconductor lasers. System for illumination 103 may also be LED or other broadband sources, provided that the light sources are sufficiently powerful to drive the photocuring process. In some instances, the system for illumination 103 inherently provide short pulses of light at the desired frequency.

Fig. 1b illustrates a schematic overview of a device comprising at least 3 functionalities: illumination and light analysis as described above, and injection of a photocurable or photoactivatable material into a cavity or body tissue 112. In this case, the applicator 105 has a lumen or one or more channels running throughout the entire length through which a fluid material can flow to the distal end (hereinafter, "tip") of the applicator. At or near the proximal end of the applicator, fluid materials can be injected via an injection subsystem 102. In combination with the applicator, the injection subsystem is disposed to cause the injected liquid material to flow into and through the applicator leaving it at its distal end where it flows into a cavity 112, onto a surface or to its final location.

Fig. 2a depicts a preferred embodiment of illumination and data analysis subsystem 113 and a cross-section of the applicator 105. A lightsource 501 with one or several excitation wavelength emits a light beam 100 which is collimated by a lens 502. The lightsource 501 can for instance consist of a laser, a LED and second laser which are combined using a dichroic mirrors. The light beam crosses a wavelength-sensitive beam-splitter 503 and is focused onto light-transmitting elements contained within the applicator 105 by a second lens 504. The so obtained actinic light is guided forward through the light-transmitting element until it exits the device from the applicator's distal end. Once actinic light interacts with a photocurable material, the obtained, back-scattered light is guided from the distal to the proximal end of the applicator where it leaves the light-transmitting element, is collimated by the lens 504 and impinges onto the beam-splitter 503. Photons having a different wavelength or any specific wavelength of interest are reflected by the beam-splitter 503 and focused by a third lens 505 to a monitoring system for data analysis 506, for example a spectral analyzer. Spectral analysis is useful for determining the change of physical and chemical status of the photocurable material. One of the advantages of the system of the present invention is the possibility to monitor the process in real time and therefore tailor the photocuring of the applied material accordingly. During the photopolymerization process brought about by actinic light, an initial low viscosity fluid material will typically have a different spectral signature after its photopolymerization during which its crosslink density or polymerization degree increase. This difference allows to discriminate between a less and a more crosslinked and polymerized material and thus to follow the kinetics of the material property changes, being its viscosity or other physical properties related to the molecular structure of the polymer or illuminated material.

Due to parallel photorheology measurements, spectral analysis allows to link chemical changes in the material to mechanical parameters thereof, thus avoiding the analysis of the mechanical properties in situ by other means such as indentation. In addition, the spectral signature or the amount of backscattered light also gives information about the position or the environment of the distal tip for instance indicate if a thick tissue segment is blocking the exiting light and thus position of the distal end has to be adjusted.

In one embodiment, the light-transmitting element consists of several optical fibers. For example, one or several optical fibers 509 can be consecrated to illumination and one or several optical fibers 508 are used to collect the light. In at least some aspects, the optical fibers of the applicator can be arranged in several ways. For instance, fibers transmitting several or certain specific wavelenghts can be envisaged or fibers of different sizes can be assembled to guide the light to the distal end of the applicator and guide it back. In a particular embodiment depicted in Fig. 2b, the light-transmitting element 509 is directly connected to the light source 501 and the spectral analyzer 506 is directly connected to the collection fibers 508.

In one embodiment, 501 consists of several light sources, of which at least one provides the actinic light to photopolymerize the injected material and at least one provides actinic light at a different wavelength to record the state of the reaction.

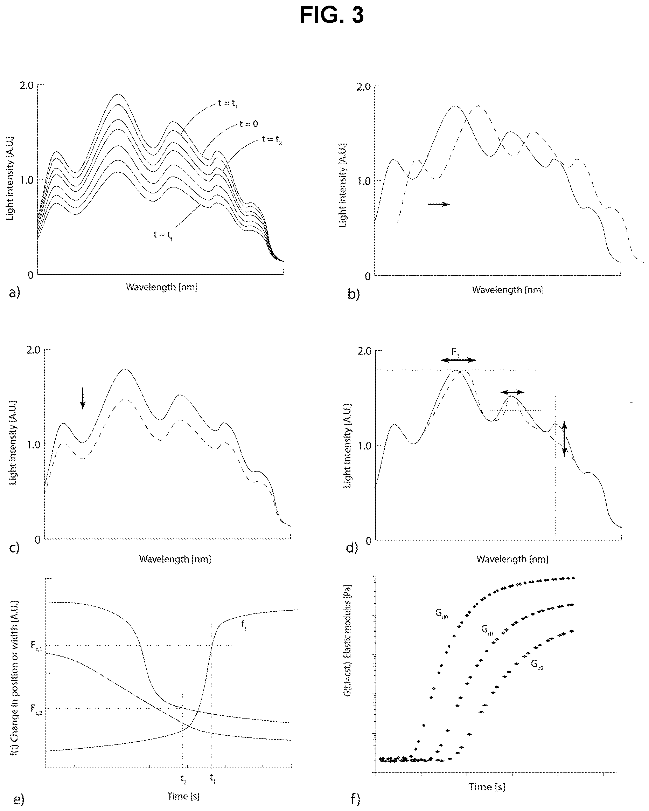

Fig. 3 shows the reflected spectrum recorded over time by a monitoring system for data analysis 506, which in one specific embodiment is a spectrometer. Using the information of the backscattered light, the spectrum gives information on the position or environment of the applicator and indicates whether the applicator is surrounded by tissue or photocurable/photocured polymeric material. Throughout photopolymerization different shifts can be observed: the spectra shifts laterally in function of wavelength or vertically in function of intensity. It is possible that the entire spectra or only one or several peaks shift over time. The change is measured by defining vertical or horizontal axes. Thus a time-intensity plot is created. The change in intensity or wavelength (F) of the spectra or the one of the peaks can be described as using a function f:

$$F = f(t)$$

t being the time. By experimental tests a critical value $F_c$ is found. Once this threshold is reached, the user interface emits a signal which indicates that the photopolymerization or chemical reaction has reached a certain degree or is completed. In addition, the information of several peaks or shifts can be evaluated at the same time to increase the precision of the monitoring for instance using reflected light around 750nm to gather information about the reaction state of material further away from the distal end while using reflected light around 550nm to access the reaction state closer to the distal end, thus:

$$F_i = f_i(t)$$

Or

$$t = f_i^{-1}(F_i)$$

i being the indices of one peak. And different functions $f_i$ and thresholds $F_{c,i}$ can indicate different states of the reaction e.g.:

$$F_{c,1} = F_1 = f_1(t_1)$$

$$F_{c,2} = F_2 = f_2(t_2)$$

If $F_1$ reaches the critical value $F_{c,1}$ a signal is emitted, in this case at $t_1$. If $F_2$ reaches the critical value $F_{c,2}$ a second signal is emitted, in this case at $t_2$. The procedure is further illustrated in Fig. 3d) and f).

This evaluation technique is based on fixing $F_{c,i}$ experimentally. The obtained plot can be subsequently combined with previously performed photorheology measurements. Photorheology measures the elastic modulus (G) of a material in function (g) of the time and total intensity of the light illumination (I):

$$G = g(t, I)$$

Thus, by combining the spectroscopy data (F,f) and the photo rheology data (G,g), the mechanical properties are evaluated online by:

$$F = f(g^{-1}(G, I))$$

or

$$G = g(f^{-1}(F), I)$$

Furthermore, by testing layers of different thicknesses F can be correlated to an elastic modulus at a certain depth ($G_d$), thus indicating the state of polymerization at a given distance (d) of the probe:

$$G_d = g_d(f^{-1}(F), I)$$

The procedure can be further generalized and applied to several peaks (indexed with i) :

$$G_{d,i} = g_{d,i}(f_i^{-1}(F_i), I)$$

For example by tacking the changes of peak #1 the elastic modulus at a distance of the tip d0 (which could be for example 5mm) is deduced:

$$G_{d0,1} = g_{d0,1}(f_1^{-1}(F_1), I)$$

Materials layers of serveal thickness (or at several depth) can be evaluated (Fig. 3f)

In case of a tissue layer blocking the exiting light the intensity Fig. 3a would suddenly increase (several orders of magnitude are possible) and the spectra would also change in function of wavelength. As certain tissues have a given reflection spectra when illuminated with light such reflections can be used to further give information (e.g. about the type of material in front of the tip - for instance tissue, blood or injected material) to the operator (e.g. surgeon) when performing an operation.

Finally, several peaks $F_1$ and $F_2$ can be compared and a critical value $F_C$ can be calculated for instance by dividing them ($F_C = F_1/F_2$) or performing any other type of mathematical calculation.

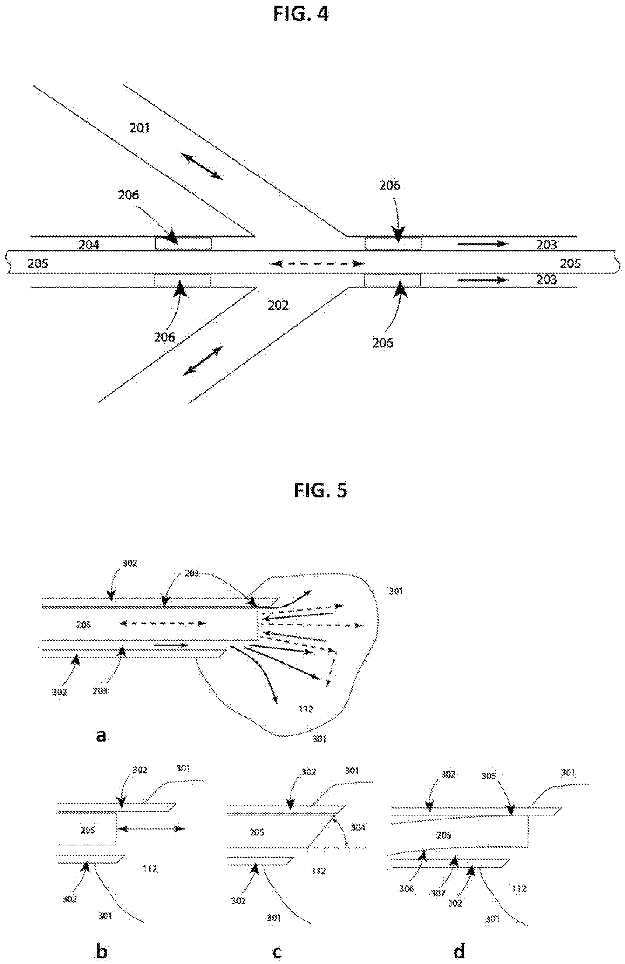

Fig. 4 is a cut view of the injection subsystem 102 according to one embodiment of the invention. Two mechanisms are responsible for combining the injection and illumination functionalities: there is a type of crossing where a fluid material can be brought close to the light transmitting element 205, along which the fluid material will flow towards the tip of the applicator. In this particular embodiment, the device consists of several injection channels (for the sake of simplicity, only two channels 201 and 202 are depicted in Fig. 4), an outflow channel 203 and a fiber channel 204. The injection channels can be aligned in an arbitrary way. The outflow channel 203 and the fiber channel 204 have to be sufficiently collinearly aligned not to over bend the light transmitting elements 205 which are typically optical fibers. The light transmitting elements 205 are inserted through the fiber channel 204 and leave the injection subsystem 102 through the outflow channel 203. The injected material can flow in both directions in the injection channels (solid arrows). The injection subsystem 102 is either an integrated part of the applicator or a separate entity connected to the applicator via the outflow and fiber channels. In either case the outflow channel 203 is connected to the distal end of the applicator where the photoactivable fluid material is ejected out of the device. In one embodiment, more than one fluid material can be delivered within the applicator through the injection channels 201, 202. These fluid materials are mixed once in the applicator, thus permitting for instance the constitution of a photocurable material starting from two or more non-photocurable materials.

The various channels of the injection subsystem may be stabilized within a housing that may be made essentially of a solid inert material and may comprise a holder to hold the device ergonomically during its use, such as for instance during surgery procedures. The light delivered by the light transmitting elements 205 is transmitted in both directions (dotted arrow), illuminating the injected material and back-propagating the light reflected or emitted by the illuminated material. Guiding elements 206 permit to align the light transmitting elements in the device while avoiding the block of the fluid flow (for example by not surrounding it completely in the radial plain).

Fig. 5 shows the mode of action of a particular embodiment of the device according to the present invention. The tip of the applicator is inserted into a tissue cavity 112 surrounded by the remaining tissue 301, such as for instance a bone cavity surrounded by bone. The cavity 112 can be closed as illustrated in fig. 5a, and can be for instance missing nucleus pulposus of an intervertebral disc, but it can also be a surface or hole such as a bone void to which the distal end of the applicator is brought to. The applicator of the device comprises a body 302 such as a solid or flexible cannula and one or more optical fibers 205 as light-transmitting elements. A fluid, photocurable material (solid arrows) pushed under an external force, flows through the outflow channels 203 which in this embodiment are interspaces between the light-transmitting element and the internal side of the applicator's body wall 302, into the cavity 112. The light (dotted arrow) guided into the optical fibers impinges onto the injected material where it is absorbed or scattered into different directions and then absorbed elsewhere. Some photons are also reflected or back-scattered and back-propagated through the light-transmitting element 205. In one embodiment, the applicator consisting of a cannula and an optical fiber, the tip of the cannula 302 and the tip of the light-transmitting element 205 can be placed at the same height or at a given distance. In one embodiment, the light-transmitting element can be inserted before or during the use of the device, and it can be moved forward (Fig. 5b, solid arrow) or backward and possibly laterally within the applicator during the procedure. In another embodiment in which the applicator comprises a cannula, the tip of the light-transmitting element 205 and the cannula can be flat or sharpened with a given angle 304 (Fig. 5c). In a particular embodiment, this angle can be different for the cannula and the fiber. In one embodiment, the light-transmitting element and the applicator's body can touch each other (305) or move freely (307), and they can have the same or a different curvature (306) (Fig. 5d). The use of other kind of tips for the light guide (e.g. implemented lens, ball lens, diffuser, conical shape, side-fire tip, transparent balloon tip, etc.) can be used.

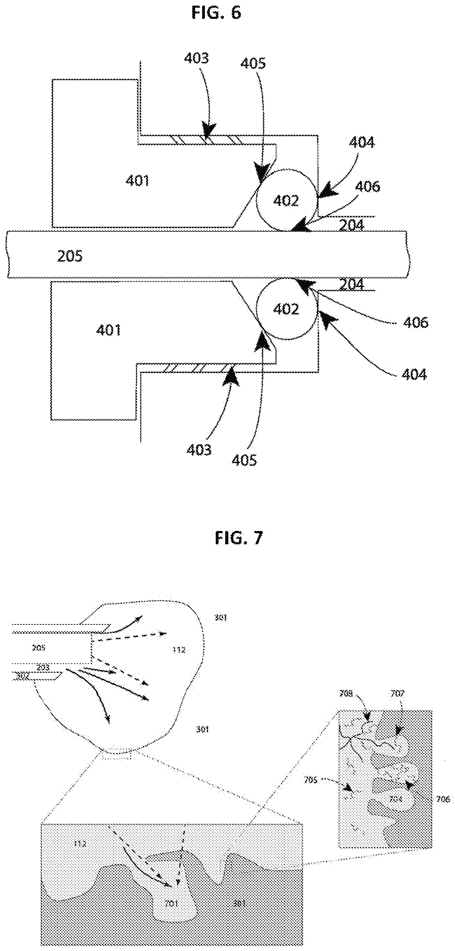

In a particular embodiment, the device according to the present disclosure comprises a back-flow locking system, as shown in Fig. 6. At the end of the fiber channel 204 a screw-tap with hole 401 is screwed into the housing of the injection subsystem. The tap has a hole in the middle to insert the light-transmitting element 205 and it can be guided by lateral joints 403. Between the screw-tap and the device a rubber ring 402 is placed. When the screw-tap is screwed into the device the ring starts to act as a valve. It deforms and locks at the same time the space between ring and device (404), ring/screw-tap (405) and ring/optical fiber (406). Thus any backward outflow through the channel 204 is inhibited and the fluid inside the device can be pressurized up to 50 bar.

Fig. 7 depicts a particular embodiment of the invention in which a fluid, non-polymerized material is directly injected into a body tissue having pores, (micro)cavities and/or voids such as the bone tissue. The tip of the applicator is put within or in close proximity with the porous or hollow tissue structure. This can be achieved through surgical meanings, such as for instance via a pre-formed bone drill wherein the applicator is inserted. The photocurable fluid, non-polymerized material flows through the interspace 203 between the optical fiber elements 205 and the internal side of the wall of the applicator under an applied pressure via e.g. an injecting system or simply a syringe. These syringes may be used to inject the fluid material, but they can also be used for mixing several materials together or increase the pressure to a given level during injection. They can be plugged in or screwed onto the device. They can be fix or removable parts of the device, specifically designed and adapted to the viscosity of the injected materials and the medical method, or they can be commercially available syringes. The pressurization and its viscosity allow the fluid, non-polymerized material to flow into macro- (701) and microscopic (704) pores of the tissue or bone 301. The pressure, preferably between 0 and 50 bar, and the viscosity, preferably between $10^{-5}$ and 1 [Pa s] or also higher, are key elements to increase the adherence of the fluid, non-polymerized material with the surrounding tissue it was injected in. Once injected, or alternatively during injection, the light can initiate the photopolymerization reaction by directly illuminating the pore 702 or also by being transmitted and scattered through the tissue 703, thus permitting the change of physical status of the fluid, non-polymerized material to a non-fluent (or solid), polymerized material. Once a polymerized material is created in the cavity, it physically blocks the macropore 701. The same type of entanglement can be created in micro-pores 704 which have a similar size of the polymer chains 705: due to its completely unpolymerized state and the applied pressure, the chains diffuse into the tissue pores and block them (706) once they solidify. Furthermore, covalent bonds (707) or other chemical bonds can be established between tissue and polymer molecules further increasing the adherence. Moreover, if a composite material containing fibers 708 is used (e.g. cellulose fibers) some of the fibers can dangle into macro- or micro-pores and once the polymer matrix around them solidifies also contribute to a higher adherence between injected material and tissue.

In at least some embodiments, the photocurable material can therefore be a filling material such as a natural or synthetic material for strengthening, replacing, healing, reinforcing or otherwise treating living tissues such us bones. Suitable filling materials include glues, epoxies, adhesives, cements, hard tissue replacement polymers, biodegradable polymers and copolymers, and various other biomaterials known in the art for strengthening, replacing or reinforcing tissue. As inert materials, bone reinforcing mixtures may be incorporated into surrounding tissue or gradually replaced by original tissue. In some embodiments, the photocurable material may be a filling material such as composite hydrogels for strengthening, replacing, healing, reinforcing or otherwise treating a nucleus pulposus of an intervertebral disc such as for example methacrylate and poly(ethylene-glycol) based polymers in combination with a photoinitiator and possibly reinforced with fibers such as cellulose nanofibrils. Those skilled in the art will recognize that numerous variants of the above mentioned materials known in the art are within the scope of the presently disclosed embodiments.

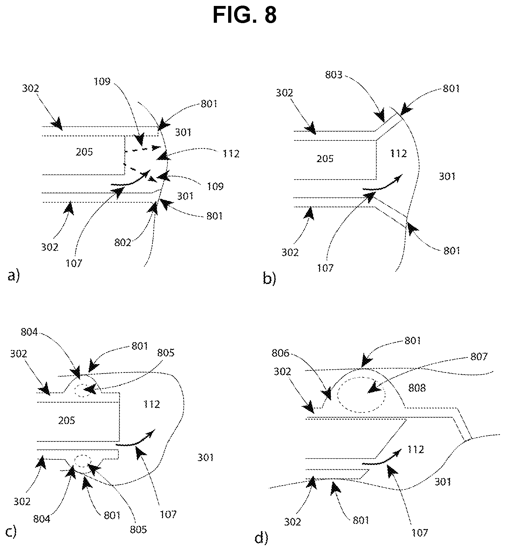

Fig. 8 A liquid can only be pressurized in a contained space. In some cases such as aneurysms, when treating the surface of a vessel or a specific area such as the surface of a joint or an organ the injected liquid might flow away from the target area. Therefore this target area has to be closed. The simplest solution for this is to press the distal end onto the tissue (Fig. 8a). Contact points 801 (or contact lines) are created and the closed cavity 112 is formed where the liquid 107 can be injected. In this case the lightguide 205 is placed not directly at the distal end but slightly inside which creates a space which can be illuminated. In one embodiment especially when the tissue 301 is a hard surface such as from a tooth, the front end of the cannula consists of a soft material (e.g. rubber or any other biocompatible and soft material) 802 which deforms when pressed against the tissue. In another embodiment (Fig. 8b) the front end of the cannula has a wide opening 803. This structure has a conic, a half-sphere or any other shape adapted to the physiology of the tissue. Such a cone can be a rigid part of the cannula. It is also imaginable that it is opened once the cannula reached its target passion for by means of a mechanical mechanism for instance similar than an umbrella or when opening a stent using a balloon. In at least one embodiment the wall of the cannula consists of a soft and highly deformable material 804 with a gas (e.g. air) or liquid (e.g. water) cavity 805 inside. In Fig. 8c is an example presented where the air or liquid cavity is a ring inside the wall of the cannula which can be filled from outside using for instance a tube to access the cavity. Thus the cannula can be inserted at a minimal diameter and once it is inside its diameter can be increased. This allows to close cavity such as an aneurysm or a blood vessel to create temporarily a closed space with a given pressure. A further example is given in fig. 8d) where only a part 806 of the otherwise rigid cannula consists of a soft material with a cavity 807 inside. Once it is pressurized the cannula is pushed in a lateral direction against the wall opposite of the tissue cavity or blood vessel.

## Claims

1. A system for

   - injecting a photosensitive material; and
   - photopolymerizing and/or activating the photosensitive material; and
   - monitoring and/or controlling the photopolymerization process, in a cavity said system comprising:
   - a light source (501) that emits actinic light;
   - an tubular applicator (105) insertable in said cavity having a proximal end, a distal end and an elongated shaft therebetween and containing at least one optical fiber (509) adapted to bidirectionally transmit light between said proximal end and said distal end,

wherein

   - said proximal end of the applicator is operably connected to the light source;
   - a light-guiding element (206) which directs light travelling from the distal end of the applicator through the at least one optical fiber towards an optical detector, said optical detector being capable of detecting the light reflected or emitted by the photosensitive material; and
   - an analysis system (113) which analyzes light coming from the photosensitive material to determine the degree of photopolymerization or activation of the photosensitive material during the photoactivation process, and said system being **characterized in that** it further comprises an injection subsystem (102) for injecting said photosensitive material through said applicator, and **in that** the distal end of the applicator (105) is arranged to inject the photosensitive material, to deliver actinic light originated from the light source to the photosensitive material and to capture light reflected or emitted by the photosensitive material.

2. The system according to claim 1 wherein the light-guiding element (206) is selected from the group of beam splitter, band-pass filter and Bragg grating.

3. The system according to claims 1 to 2, wherein the photosensitive material is an implant, filler, tissue replacement, gel or scaffold applied to a living host.

4. The system according to claims 1 to 3, wherein one or several light sources (501) emit light within a wavelength range of 200 - 3000 nanometers, preferably in a range of 200 - 700 nanometers, more preferably in a range of 315 - 700 nanometers.

5. The system according to claims 1 to 4 wherein the tubular applicator (105) has a wall and a lumen and at least one interspace between the optical fiber (509) and the internal side of the wall of said tubular element, and wherein said at least one interspace permits the delivery of the photosensitive material through the distal end of the applicator.

6. The system according to claim 5 wherein the interspace coaxially surrounds the optical fiber (509).

7. The syste according to claims 5 or 6 wherein said injection subsystem (102) introduces one or more fluids to the interspace between the optical fiber and the wall of the tubular element at or close to the applicator's proximal end, said fluids once mixed constituting the photosensitive material.

8. The system of according to claims 1 to 7, wherein the tubular applicator (105) is a needle, a cannula, a catheter or an endoscopic arm.

9. The system according to claims 1 to 8, further comprising on the applicator (105) a mechanical element such as a balloon, cone-like, semi-sphere-like or hollow element to artificially create a cavity in which a liquid material is pressurized.

10. The system according to claim 9, further comprising an element which allows to press the applicator against a surface in a controlled manner to create a cavity in which the liquid material is pressurized.

**Patentansprüche**

1. System zum:

    - Injizieren eines fotoempfindlichen Materials; und
    - Fotopolymerisieren und/oder Aktivieren des fotoempfindlichen Materials; und
    - Überwachsen und/oder Steuern des Fotopolymerisationsprozesses in einem Hohlraum,

    wobei das System Folgendes umfasst:

    - eine Lichtquelle (501), die aktinisches Licht emittiert;
    - einen röhrenförmigen Applikator (105), der in den Hohlraum einsetzbar ist und ein proximales Ende, ein distales Ende und einen länglichen Schaft dazwischen aufweist und wenigstens eine optische Faser (509) umfasst, die dazu ausgelegt ist, Licht bidirektional zwischen dem proximalen Ende und dem distalen Ende zu übertragen, wobei
    - das proximale Ende des Applikators mit der Lichtquelle wirkverbunden ist;
    - ein Lichtleitungselement (206), das Licht, das sich von dem distalen Ende des Applikators durch die wenigstens eine optische Faser bewegt, zu einem optischen Detektor hin lenkt, wobei der optische Detektor zum Detektieren des Lichts in der Lage ist, das durch das fotoempfindliche Material reflektiert oder emittiert wird; und
    - ein Analysesystem (113), das von dem fotoempfindlichen Material kommendes Licht analysiert, um den Grad einer Fotopolymerisation oder Aktivierung des fotoempfindlichen Materials während des Fotoaktivierungsprozesses zu bestimmen, und wobei das System **dadurch gekennzeichnet ist, dass** es ferner ein Injektionsuntersystem (102) zum Injizieren des fotoempfindlichen Materials durch den Applikator umfasst, und dadurch, dass das distale Ende des Applikators (105) dazu eingerichtet ist, das fotoempfindliche Material zu injizieren, aktinisches Licht, das von der Lichtquelle stammt, an das fotoempfindliche Material zu liefern und Licht zu erfassen, das durch das fotoempfindliche Material reflektiert oder emittiert wird.

2. System nach Anspruch 1, wobei das Lichtleitungselement (206) aus der Gruppe eines Strahlteilers, eines Bandpassfilters und eines Bragg-Gitters ausgewählt ist.

3. System nach Ansprüchen 1 bis 2, wobei das fotoempfindliche Material ein Implantat, Füllstoff, Gewebeersatz, Gel oder Scaffold ist, das/der in einen lebenden Wirtskörper appliziert wird.

4. System nach Ansprüchen 1 bis 3, wobei eine oder einige Lichtquellen (501) Licht innerhalb eines Wellenlängenbereichs 200 - 3000 Nanometer, bevorzugt in einem Bereich von 200 - 700 Nanometer, bevorzugter in einem Bereich von 315 - 700 Nanometer, emittieren.

5. System nach Ansprüchen 1 bis 4, wobei der röhrenförmige Applikator (105) eine Wand und ein Lumen und wenigstens einen Zwischenraum zwischen der optischen Faser (509) und der Innenseite der Wand des röhrenförmigen Elements aufweist und wobei der wenigstens eine Zwischenraum die Lieferung des fotoempfindlichen Materials durch das distale Ende des Applikators erlaubt.

6. System nach Anspruch 5, wobei der Zwischenraum die optische Faser koaxial (509) umgibt.

7. System nach Ansprüchen 5 oder 6, wobei das Injektionsuntersystem (102) ein oder mehrere Fluide in den Zwischenraum zwischen der optischen Faser und der Wand des röhrenförmigen Elements bei oder nahe dem proximalen Ende des Applikators einführt, wobei die Fluide das fotoempfindliche Material darstellen, sobald sie vermischt sind.

8. System nach Ansprüchen 1 bis 7, wobei der röhrenförmige Applikator (105) eine Nadel, eine Kanüle, ein Katheter oder ein endoskopischer Arm ist.

9. System nach Ansprüchen 1 bis 8, das ferner ein mechanisches Element, wie etwa einen Ballon, ein kegelartiges, halbkugelartiges oder hohles Element, um künstlich einen Hohlraum zu erzeugen, in dem ein flüssiges Material mit Druck beaufschlagt wird, auf dem Applikator (105) umfasst.

10. System nach Anspruch 9, das ferner ein Element umfasst, das es ermöglicht, den Applikator auf eine kontrollierte Weise gegen eine Oberfläche zu pressen, um einen Hohlraum zu erzeugen, in dem das flüssige Material mit Druck beaufschlagt wird.


**Revendications**

1. Système

    - pour injecter un matériau photosensible ; et
    - pour photopolymériser et/ou pour activer le matériau photosensible ; et
    - pour surveiller et/ou pour commander le processus de photopolymérisation dans une cavité ledit système comprenant :

        - une source de lumière (501) qui émet de la lumière actinique ;
        - un applicateur tubulaire (105) pouvant être inséré dans ladite cavité ayant une extrémité proximale, une extrémité distale et un arbre allongé entre ces dernières et contenant au moins une fibre optique (509) conçue pour transmettre de manière bidirectionnelle de la lumière entre ladite extrémité proximale et ladite extrémité distale, dans lequel
        - ladite extrémité proximale de l'applicateur est raccordée de manière fonctionnelle à la source de lumière ;
        - un élément de guidage de lumière (206) qui dirige de la lumière se propageant depuis l'extrémité distale de l'applicateur à travers la ou les fibres optiques vers un détecteur optique, ledit détecteur optique pouvant détecter la lumière réfléchie ou émise par le matériau photosensible ; et
        - un système d'analyse (113) qui analyse de la lumière provenant du matériau photosensible pour déterminer le degré de photopolymérisation ou d'activation du matériau photosensible pendant le processus de photo-activation, et ledit système étant **caractérisé en ce qu'**il comprend en outre un sous-système d'injection (102) pour injecter ledit matériau photosensible au moyen dudit applicateur, et **en ce que** l'extrémité distale de l'applicateur (105) est conçue pour injecter le matériau photosensible, pour délivrer de la lumière actinique provenant de la source de lumière au matériau photosensible et pour capturer la lumière réfléchie ou émise par le matériau photosensible.

2. Système selon la revendication 1, dans lequel l'élément de guidage de lumière (206) est sélectionné dans le groupe constitué par un diviseur de faisceau, un filtre passe-bas et un réseau de Bragg.

3. Système selon les revendications 1 à 2, dans lequel le matériau photosensible est un implant, un agent de remplissage, un tissu de remplacement, un gel ou un échafaudage appliqué à un hôte vivant.

4. Système selon les revendications 1 à 3, dans lequel une ou plusieurs sources de lumière (501) émettent de la lumière dans une gamme de longueurs d'onde allant de 200 à 3 000 nanomètres, de préférence dans une gamme allant de 200 à 700 nanomètres, de préférence encore dans une gamme allant de 315 à 700 nanomètres.

5. Système selon les revendications 1 à 4, dans lequel l'applicateur tubulaire (105) comporte une paroi et une lumière et au moins un espace intermédiaire entre la fibre optique (509) et le côté interne de la paroi dudit élément tubulaire et dans lequel ledit ou lesdits espaces intermédiaires permettent l'administration du matériau photosensible par l'extrémité distale de l'applicateur.

6. Système selon la revendication 5, dans lequel l'espace intermédiaire entoure de manière coaxiale la fibre optique (509).

7. Système selon les revendications 5 ou 6, dans lequel ledit sous-système d'injection (102) introduit un ou plusieurs fluides dans l'espace intermédiaire entre la fibre optique et la paroi de l'élément tubulaire au niveau de l'extrémité proximale de l'applicateur ou à proximité de cette dernière, lesdits fluides une fois mélangés constituant le matériau photosensible.

8. Système selon les revendication 1 à 7, dans lequel l'applicateur tubulaire (105) est une aiguille, une canule, un cathéter ou un bras endoscopique.

9. Système selon les revendications 1 à 8, comprenant en outre, sur l'applicateur (105), un élément mécanique, tel qu'un ballonnet ou un élément cunéiforme, semblable à une demi-sphère ou creux, pour créer artificiellement une cavité dans laquelle un matériau liquide est mis sous pression.

10. Système selon la revendication 9, comprenant en outre un élément qui permet de presser l'applicateur contre une surface de manière contrôlée pour créer une cavité dans laquelle un matériau liquide est mis sous pression.

## FIG. 1

a

b

## FIG. 2

a

b

# FIG. 3

## FIG. 4

## FIG. 5

a

b     c     d

## FIG. 6

## FIG. 7

# FIG. 8

**EP 3 190 986 B1**

**REFERENCES CITED IN THE DESCRIPTION**

**Patent documents cited in the description**

• US 2006240376 A